# EUROPÄISCHE PATENTSCHRIFT

(11) **EP 1 112 019 B1**
(45) Veröffentlichungstag und Bekanntmachung des Hinweises auf die Patenterteilung: **05.11.2003**
(21) Anmeldenummer: 99952413.5
(22) Anmeldetag: 16.08.1999
(51) Int. Cl.: A61B 1/005, F16C 1/14

(54) **SCHNELLVERSCHLUSS FÜR EIN ENDOSKOP**
QUICK-ACTION CLOSING ELEMENT FOR AN ENDOSCOPE
FERMETURE A ACTION RAPIDE DESTINEE A UN ENDOSCOPE

(30) Priorität: 08.09.1998 DE 19840986
(43) Veröffentlichungstag der Anmeldung: 04.07.2001
(73) Patentinhaber: XION GmbH, 13127 Berlin (DE)
(72) Erfinder: WIMMER, Viktor, Josef, D-80807 München (DE)
(74) Vertreter: Eder, Thomas, Dr.-Ing.
(86) Internationale Anmeldenummer: DE9902561
(87) Internationale Veröffentlichungsnummer: WO00013569

(56) Entgegenhaltungen:
- EP-A- 0 609 503
- JP-T3- 62 042 606
- NL-A- 279 230

## Beschreibung

Die Erfindung betrifft ein Endoskop, insbesondere flexibles Endoskop mit den Merkmalen des Oberbegriffs der Ansprüche 1 und 2.

Solche Endoskope, beispielsweise Duodenoskope, mit am Endoskopkopf üblicherweise seitlich angeordneter Optik besitzen einen Mechanismus, insbesondere einen Albarranmechanismus mit einem Albarranhebel, der es erlaubt, die durch den Arbeitskanal in das Endoskop eingeführten Sonden im Blickwinkel der Optik zu bewegen. Der Albarranhebel kann durch einen zwischen dem distalen und proximalen Ende verlaufenden Bowdenzug über einen am Endoskopgehäuse außenliegend angeordneten Betätigungshebel bewegt werden.

Bei herkömmlichen Endoskopen, wie beispielsweise in der US 4 198 959 beschrieben, ist der Albarranmechanismus bzw. der Albarranhebel fest integrierter Bestandteil des Endoskopkopfes und der Bowdenzugmantel als Kanal im Endoskopeinführschacht und -gehäuse ausgeführt, der gegenüber dem Inneren des Endoskops abgedichtet ist. Das darin verlaufende Bowdenzugseil ist üblicherweise am distalen sowie am proximalen Ende des Bowdenzugs gegenüber dem Äußeren mit Dichtringen abgedichtet. Da es sich bei diesen Dichtungen um bewegte Dichtungen handelt, durch die ein bewegbares Seil läuft, kann zumindest das distale Ende beim Gebrauch eines solchen Endoskops durch ein Nachinnenziehen eines mit beispielsweise Blut verunreinigten Seilstücks verschmutzt werden. Derart verunreinigte Endoskope bzw. Endoskopköpfe sind schwer zu reinigen und bedingen bei erneutem Gebrauch Hygieneprobleme bzw. Verschleppung von Keimen, sowie eine zunehmende Schwergängigkeit des Bowdenzugs bzw. der gesamten Albarranhebelbetätigungsmechanik.

Bei einem anderen Endoskoptyp kann der Bowdenzug durch eine am proximalen Ende befindliche Öffnung für eine Spritze zu Reinigungszwecken gespült werden. Die Flüssigkeit kann beim Spülen am distalen Ende des Bowdenzugs, das nicht abgedichtet ist, austreten. Bei unsachgemäßer Vorgehensweise kann hierbei allerdings durch einen für die inneren Dichtungen zu hohen Druck Spülflüssigkeit ins Endoskopinnere gelangen. Solche in der Praxis aufgetretenen Fälle resultieren meist aus bereits vorhandenen Verschmutzungen, die eine Verringerung des Querschnitts des zu spülenden Kanals und somit eine geringere Durchflußgeschwindigkeit bewirken, wobei der maximal erlaubte Druck durch ein zu starkes Drücken der Spritze durch das Bedienpersonal leicht überschritten werden kann. Die Folge ist dann eine ebenso zeitaufwendige wie kostspielige Reparatur des Endoskops.

Beide bekannten Typen beinhalten des weiteren den gemeinsamen Nachteil, daß der Albarranmechanismus durch seine integrierte Bauweise nur schwer reinigbar ist. Ein hierfür in der Praxis erforderliches Putzen mit einer kleiner Bürste zeigte, daß der empfindliche Albarranmechanismus des öfteren beschädigt wird. Eine erforderliche Reparatur des Albarranmechanismus ist durch die integrierte Bauweise zudem kostspielig und zeitaufwendig, da der gesamte gekapselte Kopf, der auch die Optik beinhaltet, freigelegt werden muß und nach erfolgter Reparatur des Albarranmechanismus die Verkapselung wiederhergestellt werden muß.

Bei einem weiteren aus der JP Sho 62 42 606 bekannten Endoskop kann, nachdem das Bowdenzugseil am proximalen Ende durch Lösen einer Schraubverbindung frei ist, der Albarranmechanismus vom Endoskopkopf abgeschraubt und abgenommen werden. Nach dem Lösen einer distalen Anschlagplatte mittels Schrauben kann der Bowdenzugmantel herausgezogen werden.

Diese Art der Lösung der Befestigung bedarf allerdings eines Technikers, üblicherweise aus Haftungsgründen sogar eines Wartungstechnikers der Herstellerfirma des Endoskops, da ein derartiges aufwendiges Lösen der Verbindungen einer Bedienperson, beispielsweise einer Arzthelferin, weder zugemutet noch von ihr ausgefiihrt werden kann.

Sofern dieses Endoskop keine Dichtungen gegen das Eindringen vom Verunreinigungen am distalen Ende besitzen sollte, müßte das Endoskop nach jedem Gebrauch durch Lösen aller oben beschriebener Verbindungen gereinigt werden. Da hierfür ein Techniker bemüht werden muß, ist diese Reinigungsart kompliziert, kostenintensiv und zeitaufwendig und deshalb im medizinischen Alltag nicht praktikabel.

Dieses Problem wurde durch ein Endoskop nach der DE 196 27 016 C1 gelöst, indem eine Trägervorrichtung für den Albarranmechanismus durch das Lösen einer Schnellspannvorrichtung für das Bowdenzugseil von einer Bedienperson in Längsrichtung vom Endoskopkopf abgenommen werden kann und hierdurch sowohl die Trägervorrichtung, das Bowdenzugseil, der Bowdenzugmantel als auch der Bowdenzugkanal auf einfache Weise durch das Bedienpersonal gereinigt werden kann.

In der Praxis hat sich aber gezeigt, dass eine, im bekannten Stand der Technik nicht existierende Schnellspannvorrichtung, schwer realisierbar ist, da die geforderte Genauigkeit bei der Bewegung eines am distalen Ende befindlichen Mechanismus einen unabhängig von der Zahl der Lös- und Befestigungsvorgängen immer gleichbleibenden Arbeitsbereich des distalen Mechanismus und der Betätigungmechanik am proximalen Ende hierfür erfordert.

Der vorliegenden Erfindung liegt daher die Aufgabe zugrunde, ein Endoskop mit einer Schnellspannvorrichtung zu schaffen, welche die geforderte Genauigkeit bei der Bewegung eines am distalen Ende befindlichen Mechanismus unabhängig von der Zahl der Lös- und Befestigungsvorgänge inclusive dem Abnehmen des distalen Mechanismus und Herausziehen des Bowdenzugseils und -mantels durch eine Bedienperson und eine einfache Lösbarkeit des am distalen Ende befindlichen Mechanismus zu Reinigungszwecken durch eine Bedienperson gewährleistet.

Weiterhin liegt der Erfindung die Aufgabe zugrunde, ein Endoskop mit einer Schnellspannvorrichtung zu schaffen, das auch bei einer sehr verschlungenen, stark abgewinkelten Endoskopschaftstellung die Funktionalität des am distalen Ende angeordneten, durch einen Bowdenzug betätigbaren Mechanismus gewährleistet.

Diese Aufgabe wird erfindungsgemäß durch die Merkmale der Ansprüche 1 und 2 gelöst.

Durch die Ausbildung einer Schnellspannvorrichtung in Form eines Schnellverschlusses mit einer Feststellposition für das Lösen und Befestigen eines Bowdenzugseils mittels einer Befestigungseinrichtung wird gewährleistet, dass das Befestigen in einer immer gleichbleibenden exakten Position bezüglich der Längsverschiebung des Bowdenzugseils erfolgt. Hierdurch bleibt der Arbeitsbereich für die gesamte Betätigungsmechanik (proximal sowie distal) für einen zu betätigenden Mechanismus, insbesondere Albarranmechanismus, am distalen Ende unanhängig von der Anzahl der vorangegangenen Befestigungs- bzw. Lösvorgänge gleich.

Zudem kann die Befestigungseinrichtung im Schnellverschluss gelöst werden und in einer hiervon unterschiedlichen Position bzw. Feststellposition geschlossen werden. Hierdurch kann während eines Arbeitsvorgangs eine Art Nachfassen des Bowdenzugseils in beide Richtungen erfolgen und vorteilhafterweise auf durch Extremsituationen, wie stark verschlungener, stark abgewinkelten Endoskopschaft, veränderte Arbeitsbereiche reagiert werden.

Gleichzeitig ist durch die Ausbildung eines zweiten Schnellverschlusses das einfache Abnehmen des am distalen Ende befindlichen Mechanismus und Herausziehen des Bowdenzugseils durch eine Bedienperson möglich. Hierdurch kann auf einfache Weise die nach jeder Verwendung des Endoskops notwendige Reinigung durch das Bedienpersonal erfolgen. Das Endoskop kann hierfür durch das Lösen des ersten und zweiten Schnellverschlusses schnell und einfach in die Bestandteile Bowdenzugseil, distaler Mechanismus zerlegt werden, wobei wegen der nicht notwendigen Dichtungen alle verunreinigten Teile sowie der Innenraum des Bowdenzugmantels auf einfache Weise vom Bedienpersonal selbst, beispielsweise mittels effektiver Reinigungs- und evtl. auch Sterilisationsmethoden, wie z.B. Ultraschall, Autoklav usw., gereinigt werden können.

Zudem kann der Albarranmechanismus bei Verschleiß, Beschädigung etc. auf einfache Art und Weise durch einen neuen Albarranmechanismus, evtl. auch gegen Weiterentwicklungen oder andere Typen, vom Bedienpersonal ausgetauscht werden, ohne daß ein zeitaufwendiges und kostspieliges Zerlegen des Endoskopkopfes und des proximalen Endoskopendes durch einen Techniker nötig wäre.

Ist im Inneren des Endoskops, wie in der DE 196 27 016 C1 beschrieben, ein eigener Bowdenzugkanal ausgebildet, kann zusätzlich der Bowdenzugmantel ebenso wie das Bowdenzugseil hierfür zumindest ein Stück oder ganz herausgezogen werden. Hierdurch wird die Lösbarkeit des Albarranmechanismus bzw. der Trägervorrichtung und damit die Reinigung des Bowdenzugs weiter erleichtert und verbessert, da nun bei einer lösbaren Verbindung für das Bowdenzugseil am proximalen Ende in Form eines ersten Schnellverschlusses der gesamte Bowdenzug, also Bowdenzugseil und -mantel, zusammen oder einzeln nacheinander um zumindest ein Stück oder gar komplett herausgezogen werden kann.

Hierbei ist es vorteilhaft, wenn wie oben beschrieben, der Bowdenzugmantel nicht am proximalen und/oder distalen Ende, beispielsweise mittels einer Schraubverbindung, mit dem Endoskop ortsfest, obwohl grundsätzlich möglich, verbunden ist, sondern nur jeweils über einen proximalen und einen distalen Anschlag in der Bewegung in Längsrichtung begrenzt ist. Hierbei kann der distale Anschlag durch die entnehmbare Trägervorrichtung gebildet werden, so dass nach dem Lösen des Bowdenzugseils durch den ersten Schnellverschluss und nach dem Abnehmen der Trägervorrichtung durch Lösen des zweiten Schnellverschlusses nicht nur das Bowdenzugseil sondern auch der Bowdenzugmantel am distalen Ende in Längsrichtung herausgezogen werden kann.

In einer Ausführungsform der Erfindung befindet sich der Albarranmechanismus, in einer Trägervorrichtung, die beispielsweise in Form eines Schlitten ausgebildet sein kann. Dieser Schlitten weist Führungselemente auf, die im Zusammenwirken mit komplementären Elementen am Endoskopkopf eine seitliche Bewegung, also senkrecht zur Bewegungsrichtung des Bowdenzugseils, verhindern. Diese Führungselemente können beispielsweise als zylinderförmige Fortsätze, die in entsprechende Ausnehmungen am Endoskopkopf in Längsrichtung, also der Bewegungsrichtung des Bowdenzugseils eingreifen, ausgebildet sein. Hierbei kann es sich bei diesen Fortsätzen auch um hohlzylinderförmige Fortsetzungen der in einem Endokopkopf vorhandenen Kanäle, wie Bowdenzugkanal und/oder Arbeitskanal für einzuführende Geräte, handeln, die in entsprechend im Innenumfang vergrößerte Außnehmungen im distalen Endbereich der Kanäle eingreifen. Selbstverständlich sind auch seitliche in Längsrichtung verlaufende Führungen, wie beispielsweise eine Schwalbenschwanzführung, denkbar. Hierdurch wird vorteilhafterweise ein fehlerhaftes Einsetzen und dadurch enstehende Beschädigungen durch eine Bedienperson verhindert, da die Einsetzrichtung, die wegen des relativ starren Bowdenzugseils im wesentlichen in Längsrichtung erfolgen muß, hierdurch vorgegeben ist.

In weiterer Ausgestaltung der Erfindung wird sowohl die Bewegung der Trägervorrichtung als auch des Bowdenzugmantels in Längsrichtung durch den zweiten Schnellverschluss, beispielsweise in Form eines Schnapp- oder Rastverschlusses, nach dem Einsetzen verhindert. Hierdurch kann auch bei einem Bowdenzugmantel der nur in Form von proximalen und distalen Anschlägen in Längsrichtung fixiert bzw. ortsfest mit dem Endoskop verbunden ist, eine exakte Position des Bowdenzugseils nach dem Einsetzen erreicht werden, wobei sich der Albarranmechanismus bzw. der Albarranhebel in seiner Ruheposition befindet. Diese wird beim Einführen des Bowdenzugseils in den Bowdenzugmantel automatisch durch den hierbei entstehenden Gegendruck in Richtung des distalen Endes erreicht, kann dabei aber auch durch eine entsprechende Rückholfeder am Albarranhebel unterstützt werden.

Diese Ausführungsform der Erfindung gewährleistet zusätzlich zur besseren Lösbarkeit des Albarranmechanismus bzw. der Trägervorrichtung und den oben genannten daraus resultierenden Vorteilen auch eine leichtere Reinigung des Bowdenzugs bzw. des -kanals und somit der sonst nur sehr schwer hygienisch rein und steril zu haltenden Bereiche durch das Bedienpersonal, die aus Hygienegründen nach jedem Einsatz des Endoskops erfolgen muss.

In weiterer Ausgestaltung ist das proximale Ende des Bowdenzugseils beispielsweise mittels Löten oder Hartlöten versteift, ohne dass sich dessen Außenumfang vergrößert. Hierdurch kann vorteilhafterweise eine Beeinträchtigung der Befestigungswirkung im ersten Schnellverschluss, beispielsweise verursacht durch Quetschung, Zerfaserung o.a Zerstörung des Seilendes, durch Erhöhung der Druckfestigkeit vermieden werden, ohne dass eine für das Einführen des Bowdenzugseils vom distalen Ende her unerwünschte Vergrößerung des Innendurchmessers des Bowdenzugmantels und hiermit auch eines eventuell vorhandenen zusätzlichen Bowdenzugkanals notwendig wäre.

In einer vorteilhaften Ausführungsform der Erfindung kann die arretierte Feststellposition und das Lösen bzw. das Verbinden des Bowdenzugseils mit dem ersten Schnellverschluss mittels eines einzigen Bedienelements gleichzeitig ausgeführt werden. Das Bedienelement ist hierfür beispielsweise als Drehknopf ausgebildet, der für das Arretieren eine in Längsrichtung wirkende Führungscharakteristik und in einer Ebene senkrecht zu dieser Richtung ein auf das Seil einwirkenden Befestigungsmechanismus aufweist. Der Befestigungsmechanismus kann dabei als ein in dieser Ebende drehbarer Exzenter oder Nocken ausgebildet sein, der direkt oder über einen mittels Federkraft vorgespannten Hebel auf das proximale Ende des Bowdenzugseils einwirkt. Die Führungscharakteristik kann dabei beispielsweise als eine am Außenumfang verlaufende in Drehrichtung zur Feststellposition sich verjüngende Ausnehmung, die mit einem ortsfest am Endoskop angeordneter Zapfen zusammenwirkt, ausgebildet sein. Die Endposition "Arbeitsbereich" in entgegengesetzter Drehrichtung wird dagegen durch einen diese Ausnehmung in Längsrichtung erweitertenden Durchbruch gebildet, so dass ein ortsfest am Endoskop angeordneter Zapfen durch diesen Durchbruch in die Führungscharakteristik ein- bzw. ausgeschwenkt werden kann.

In einer besonders vorteilhaften Ausgestaltung der Erfindung kann der Schnellverschluss, der beispielsweise als schwenkbare Klappe ausgebildet ist, in einer Richtung entgegengesetzt zum distalen Ende über den Arbeitsbereich hinaus geschwenkt werden, wodurch ein Durchrutschen des Bowdenzugseils in der Befestigungseinrichtung um die Länge dieses Herausschwenkens erfolgt.

Dies kann bei einer sehr verschlungenen, stark abgewinkelten Endoskopschaftstellung und der daraus resultierenden fehlerhaften Rückstellwirkung des Albarranmechanismus in Ruhestellung dazu dienen, mehr Vorspannung in Schubrichtung in den Bowdenzug einzubringen. Hierdurch kann vorteilhafterweise der bei bekannten Endoskopen in dieser Stellung verminderte Arbeitsbereich ausgeglichen werden, so dass die Funktion bzw. die Beweglichkeit des Albarranmechanismus auch in einer solchen Lage nicht eingeschränkt ist.

Erfolgt das Schwenken des außenliegenden oder zumindest von Außen zugänglichen Schnellverschlusses in Längsrichtung des Bowdenzugseils, also ein reines Verschieben, so wird das Bowdenzugseilende vorteilhafterweise nicht zusätzlich auf Biegung beansprucht. Hierdurch kann einem eventuell möglichen Brechen des Bowdenzugseilendes, insbesondere eines versteiften Endes, vorgebeugt werden.

Ist der Schnellverschluss als eine in Längsrichtung des Bowdenzugseils verschiebbare Spannvorrichtung ausgebildet, beispielsweise in Form eines Schlittens mit einer mittels eines Spannelements spannbaren bzw. öffen- und schließbaren Spannzange, wird das Bowdenzugseilende in einem innerhalb der Spannzange befindlichen Bereich von mehreren um den Umfang verteilten Seiten oder über den gesamten Umfang herum von einer nach Innen wirkenden Kraft beaufschlagt bzw. eingeklemmt. Hierdurch kann vorteilhafterweise einer Schädigung durch Deformierung oder Quetschung eines zu kleinen Bereiches oder Punktes und/oder einer einseitigen Deformation des Querschnitts des Bowdenzugseils vorgebeugt werden.

Allen Ausführungsformen nach der Erfindung sind die Vorteile wie Einbringen einer Vorspannung, also Veränderung des Arbeitsbereiches sowie die verbesserten Reinigungsmöglichkeiten durch ein Lösen und etwaiges Zerlegen der Schnellverschlüsse, Entnehmen des Endoskopkopfes und/oder Herausziehen des Bowdenzugs und dessen etwaige Zerlegung, bis hin zu einer kompletten Zerlegung dieser Teile durch eine Bedienperson gemein. Weiterhin können hierdurch Wartung, Reparatur, Austausch einzelner Teile, die bisher nur durch einen Techniker möglich war, in einfachster Weise von einer Bedienperson selbst erledigt werden.

Weitere vorteilhafte Ausführungsformen der Erfindung ergeben sich aus den Unteransprüchen.

Die Erfindung wird nachfolgend anhand in den Zeichnungen dargestellter Ausführungsbeispiele näher erläutert. In den Zeichnungen zeigen:
- Fig. 1: eine Seitenansicht eines proximalen Endes eines Endoskops mit einem Schnellverschluss;
- Fig. 2: eine vergrößerte Darstellung nach Fig. 1 mit dem Schnellverschluss in verschiedenen Positionen;
- Fig. 3: eine Rückansicht nach Fig. 1;
- Fig. 4: eine Einzeldarstellung einer Klappe eines Schnellverschlusses ohne Hebelmechanismus in einer Ansicht X nach Fig. 3;
- Fig. 5a -e: verschiedene Ansichten eines Drehknopfes eines Schnellverschlusses;
- Fig. 6: eine perspektivische Ansicht eines distalen Endes eines Endoskops;
- Fig. 7: das distale Ende eines Endoskops nach Fig. 6 mit abgenommener Trägervorrichtung;
- Fig. 8: eine Seitenansicht eines proximalen Endes eines Endoskops mit einer anderen Ausführungsform eines Schnellverschlusses in teilgebrochener Darstellung;
- Fig. 9: eine Seitenansicht eines proximalen Endes eines Endoskops nach Fig. 8 ohne Steuermechanik;
- Fig. 10: eine Schnittansicht entlang der Linie I - I' in Fig. 8 und
- Fig. 11: eine Seitenansicht eines proximalen Endes eines Endoskops nach Fig. 8 mit aufgesetzter Spülkappe.

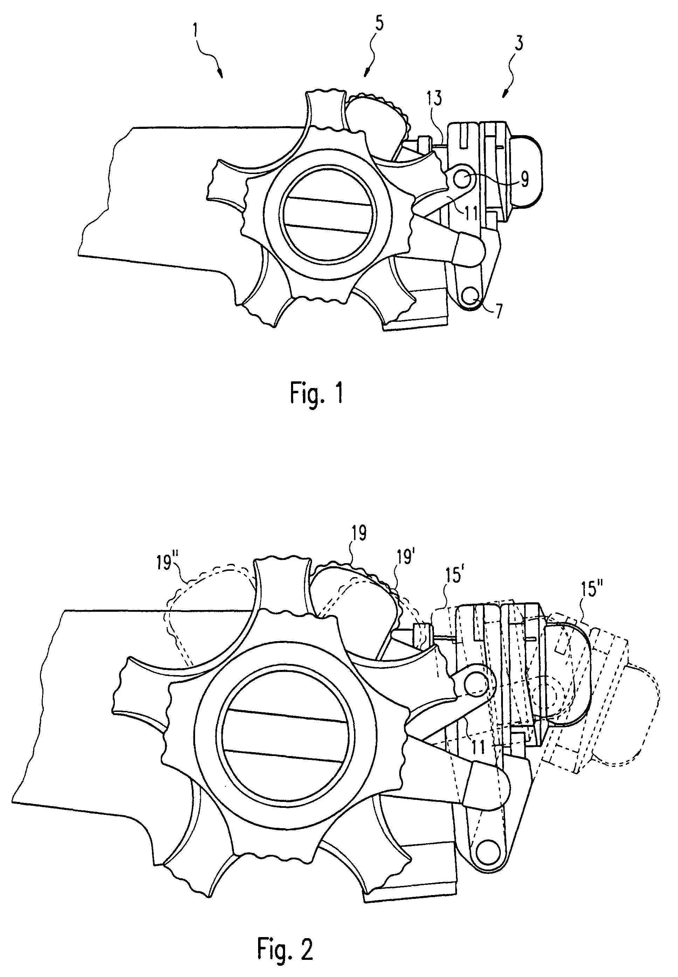

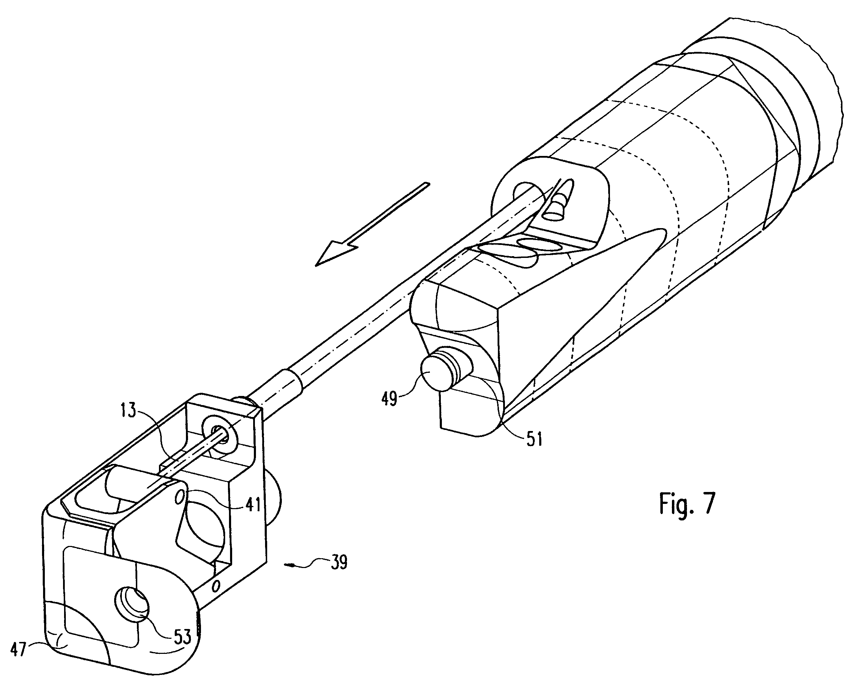

Das in Fig. 1 dargestellte proximale Ende 1 eines Endoskops, insbesondere eines Duodendoskops mit flexiblem Endoskopschaft, weist an seinem stirnseitigen Ende einen Schnellverschluss 3, welcher in seinem unteren Bereich über eine Achse 7, beispielsweise in Form einer durchgehenden Welle oder zweier einzelner nicht durchgehender Bolzen oder Schrauben, an dem Endoskopgehäuse schwenkbar gelagert ist, auf. Dieser in Form einer schwenkbaren Klappe ausgebildete Schnellverschluss 3 kann mittels eines Hebelmechanismus welcher im oberen Bereich des Schnellverschlusses 3 mit diesem schwenkbar verbunden ist um die senkrecht zur Zeichenebene stehende Drehachse 7 zwischen einer Anfangs- und Endposition geschwenkt werden. Auf diese Weise wird ein im oberen Bereich des Schnellverschlusses befestigtes Bowdenzugseil 13 eines zum distalen Ende führenden Bowdenzugs in seiner Längsrichtung senkrecht zur Achse 7 verschoben, um einen am distalen Ende angeordneten, mit dem Bowdenzugseil verbundenen Mechanismus, beispielsweise einem Albarranhebel 41 (Fig. 7), von seiner Ruheposition in eine Arbeitsposition zu schwenken.

Die Schwenkbewegung des Schnellverschlusses von einer Endposition 15' in eine andere Endposition 15" ist in Fig. 2 gegenüber einer wie auch in Fig. 1 normal dargestellten Feststellposition jeweils gestrichelt dargestellt. Hierbei kann ein im Bereich einer Steuermechanik 5 für die Bewegung des flexiblen Schachtes ortsfest und drehbar am Endoskopgehäuse um eine Achse senkrecht zur Zeichenebene angeordneter Betätigungshebel 19, welcher mit dem ebenfalls in einer Achse senkrecht zur Zeichenebene am Schnellverschluss 3 drehbar gelagerten Hebel 11 schwenkbar verbunden ist, von einer Bedienperson zur Erreichung der Endposition 15', 15" des Schnellverschlusses von einer Stellung 19' bis zu einer Stellung 19" bewegt werden.

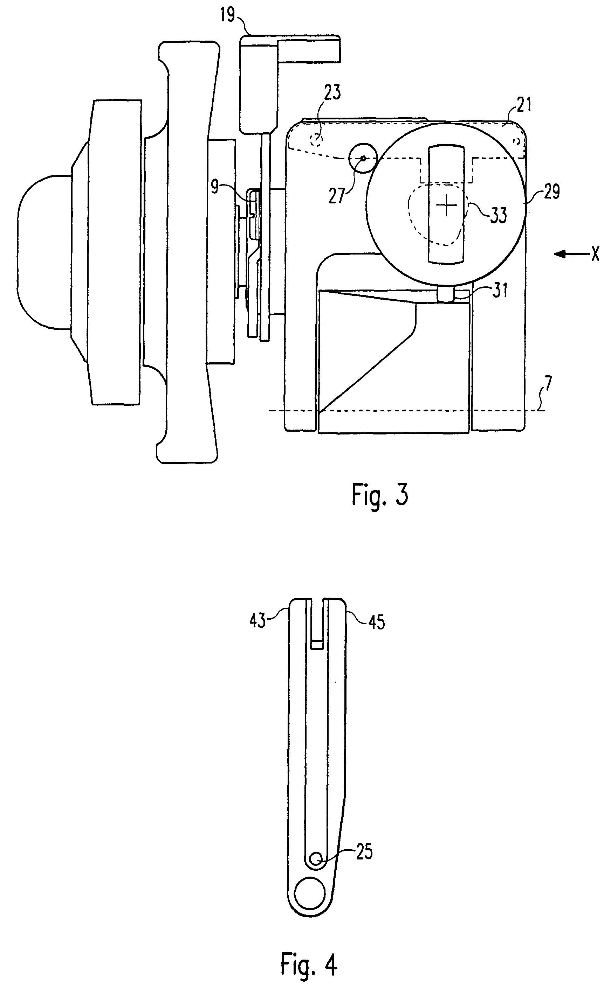

Wie aus Fig. 3 ersichtlich, erfolgt die Befestigung bzw. das Lösen eines proximalen Endes des Bowdenzugseils 13 über einen im oberen Bereich des Schnellverschlusses 3 befindlichen Hebelmechanismus. Dieser Hebelmechanismus besteht aus einem T-förmigen Hebel 21, welcher an seinem einen Endpunkt seines waagrechten Schenkels mittels einer Welle oder Schraube 23 in der Zeichenebene um eine Achse senkrecht zu dieser Ebene schwenkbar gelagert ist. An seinem gegenüberliegenden Endpunkt seines waagrechten Schenkels greift in nicht näher dargestellter Weise eine Rückstellfeder ein, deren anderes Ende mit einem im Bereich unterhalb dieses Hebels liegenden Punkt 25 des Schnellverschlusses 3 ortsfest mit diesem verbunden ist (siehe Fig. 4).

Wie in Fig. 3 dargestellt befindet sich im Schnellverschluss 3 ein Druchbruch bzw. Loch 27 für das proximale Ende des Bowdenzugseils 13 im engeren Bereich des Drehpunktes 23 des Hebels 21, so dass der senkrecht zur Zeichenebene liegende Durchbruch 27 von einer Unterkante des Hebels 21 im näheren Bereich des Drehpunkts 23 geschnitten wird. Die direkte Verbindungsstrecke zwischen Drehpunkt 23 und Loch 27 weist hierbei eine im Verhältnis zur Verbindungsstrecke zwischen Drehpunkt 23 und Angriffspunkt der Rückstellfeder wesentlich geringere Länge auf, so dass eine Hebelwirkung entsteht. Auf diese Weise wird auf ein eingesetztes Ende eines Bowdenzugseils 13 über diese Hebelwirkung eine gegenüber der Rückstellkraft einer am anderen Ende angeordneten Feder vergrößerte Kraft ausgeübt, so dass das Bowdenzugseil 13 im Druchbruch 27 durch den Hebel 21 im Zusammenwirken mit dem in einer Achse liegenden Durchbruch 27 durch eine Vorderwand 43 und Rückwand 45 (Fig. 4) der Klappe festgeklemmt wird. Selbstverständlich ist es auch denkbar, dass der Hebel 21, statt wie im Ausführungsbeispiel zwischen der Vorder- und Rückwand 43, 45 der Klappe, außen an der Klappe angeordnet ist. Allerdings vermindert sich dann die Klemmwirkung auf das Bowdenzugseil 13, da dieses dann nicht mehr an zwei Übergängen, nämlich Rückwand 45 zu Hebel 21 und Hebel 21 zu Vorderwand 43, sondern nur nach an einem Übergang eingeklemmt wird.

Die Schwenkbewegung des Hebels 21 erfolgt durch einen senkrecht zur Zeichenebene drehbar im oberen Bereich des Schnellverschlusses unterhalb des Hebels 21 drehbar gelagerten Bedienelements in Form eines Drehknopfes 29, welcher auf seiner senkrecht zur Zeichenebene in Fig. 3 stehenden Drehachse im Bereich des Hebels 21 einen Exzenter bzw. einen Nocken 33 aufweist, welcher ortsfest auf der Welle des Drehknopfes 29 sitzt. Dieser Nocken 33 steht mit seiner Umfangsfläche mit dem stimseitigen unteren Ende des Hebels 21, also dessen senkrechten Schenkels, in Verbindung, so dass bei einer Drehung des Nockens 33 über den Drehknopf 29 der Hebel 21 in der Zeichenebene (Fig. 3) von einer untersten bis zu einer obersten Position geschwenkt werden kann.

In der in Fig. 3 dargestellten untersten Position, in der der Nocken 33 mit seinem kleinsten Radius am Hebel 21 angreift, wird der Druchbruch 27 vom Hebel 21 überdeckt und das Bowdenzugseil 13 hierdurch wie beschrieben festgeklemmt. Hierbei ist es auch denkbar, dass der Nocken 31 nicht mehr mit seiner Umfangsfläche an der unteren Stirnseite des Hebels 21 anliegt. Wird der Nocken 31 über den Drehknopf 29 dagegen in eine Stellung bewegt, so dass eine Umfangsfläche mit größeren oder gar größtem Radius an der unteren Stirnseite des Hebels 21 angreift, wird der Hebel 21 aus der beschriebenen Position ausgelenkt und hierdurch der Durchbruch 27 freigegeben, so dass das proximale Ende eines Bowdenzugseils 13 in diesen eingeführt, bzw. aus diesem herausgezogen werden kann.

Sowohl das Befestigen als auch das Festklemmen des Bowdenzugseils 13 kann in der in Fig. 1 dargestellten Position des Schnellverschlusses 3 erfolgen. Um diese Position sicher und ortsfest zu gewährleisten, befindet sich am Außenumfang des Drehknopfes 29 eine sich in ihrer Dicke bezüglich der Drehachse zu einem Endpunkt hin verjüngende Ausnehmung gleichbleibender Tiefe in Form einer Schnekke 35. An ihrem Anfangspunkt weist diese Schnecke einen Eingang in Form eines Durchbruchs auf, so daß sie zur Unterseite des Drehknopfs 29 geöffnet ist.

Die Dicke dieser Schnecke 35 entspricht an ihrer dünnsten Endposition in etwa dem Durchmesser eines am Gehäuse des Endoskops ortsfest angeordneten Zapfens 31, welcher in die Schnecke in ihrer Ebene, also der Zeichenebene nach Fig. 3, in einer Feststellposition des Schnellverschlusses 3 eingreift.

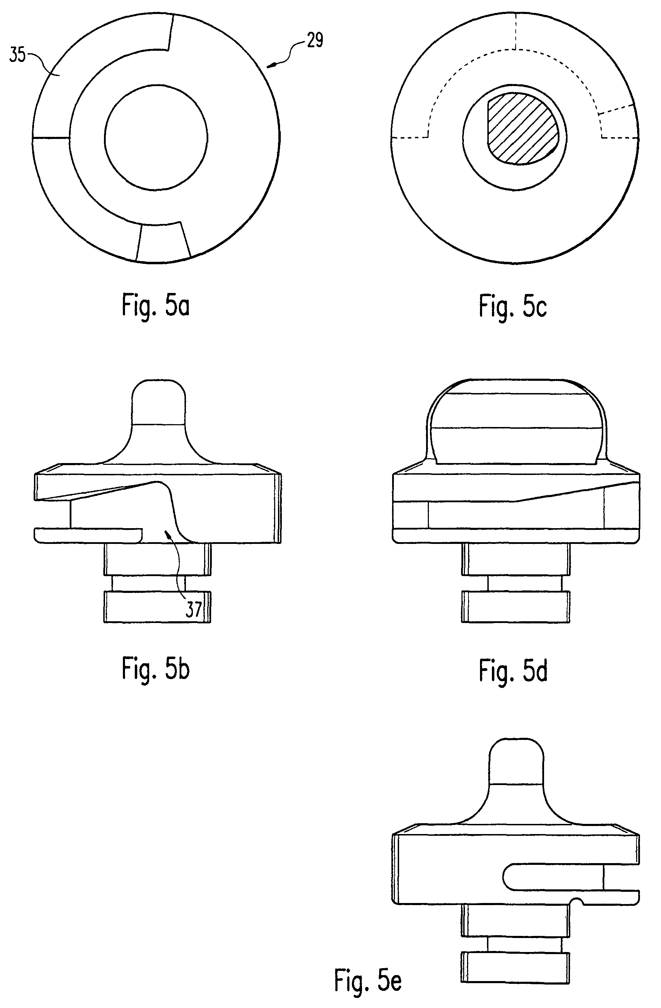

Die Öffnung 37 der Schnecke 35 ist dagegen etwas größer als der Durchmesser des Zapfens 31 ausgebildet, um diesen sicher in das Schneckenprofil einführen zu können und in einer Arbeitsposition des Drehknopfes 29 mit festgeklemmten Bowdenzugseil 13 eine Bewegung der Klappe zwischen den Endpositionen 15' und 15" zu gewährleisten. Hierbei kann wie in Fig. 5b ersichtlich die Eingangsöffnung zur Unterseite des Drehknopfes 29 hin erweitert sein, um das Einführen des Zapfens 31 zu erleichtern.

Das Schneckenprofil erstreckt sich, wie aus Fig. 5a und Fig. 5c ersichtlich, über einen 180° Bereich des Außenumfangs des Drehknopfes 29, wobei auch andere Bereiche, beispielsweise von 60° bis zu 360°, denkbar sind. Die Größe des Bereichs ist hierbei nur von dem Zusammenwirken mit der Funktion des Nockens 33 und eventuell zusätzlich vom Bedienkomfort abhängig. Je nach Eingriffspunkt des Zapfens 31 in den Eingang des Schneckenprofils muß der Nocken mit seinem kleinsten Radius an der unteren Stirnseite des Hebels 21 angreifen, um das Ende des Bowdenzugseils 13 festzuklemmen und ein freies Schwenken des Schnellverschlusses 3 zu ermöglichen. Am Eingriffspunkt des Zapfens 31 in die dünnste Stelle des Nockenprofils muß der Nocken 33 dagegen mit seinem größten Radius an der unteren Stirnseite des Hebels 21 angreifen, um bei einem Arretieren des Schnellverschlusses in seiner Feststellposition (Fig. 1) insbesondere das Einführen und Festklemmen des Seils 13 an einer vorbestimmten gleichbleibenden Stelle zu ermöglichen. Hierfür kann die Schnecke, wie in Fig. 5a dargestellt, einen größeren Bereich, beispielsweise 90°, kleinster Dicke aufweisen, innerhalb dessen das Freigeben bis zum Festklemmen des Seils 13 über den Nocken 31 und dem Hebel 31 erfolgt, wobei der Schnellverschluss 13 durch das Eingreifen des Zapfens 31 in diesen Bereich ohne Spiel in seiner Feststellposition fixiert bzw. arretiert ist.

Hierdurch kann vorteilhafterweise eine Fehlbedienung, wie Festklemmen des Bowdenzugseils 13 in einer falschen Stellung des Schnellverschlusses 3 und ein daraus resultierender eingeschränkter oder falscher Arbeitsbereich für den über den Bowdenzug zu betätigenden Mechanismus am distalen Ende des Endoskops, beispielsweise ein Albarranhebel 41, vermieden werden.

Wie aus Fig. 2 ersichtlich, stimmt die vordefinierte Feststellposition des Schnellverschlusses im Ausführungsbeispiel nicht mit dessen Endposition 15' überein, sondern in einer von dieser Endposition 15' etwas entfernten Stellung (Fig. 1). Hierdurch kann in den Bowdenzug eine kleine Vorspannung eingebracht werden, um beispielsweise ein vorhandenes geringes Spiel zwischen Bowdenzugseil 13 und Bowdenzugmantel auszugleichen, da sonst in Schubrichtung eine Endposition des Mechanismus am distalen Ende möglicherweise nicht erreicht werden kann. Die daraus resultierende größere Länge des Bowdenzugseils 13 im Bowdenzug kann in der anderen Endposition 15", also in Zugrichtung, durch deren geringfügigen Verschiebung in Zugrichtung und dem dadurch geringfügig erweiterten Arbeitsbereich ausgeglichen werden.

In manchen Anwendungsfällen muß der flexible Endoskopschaft derart gebogen und/oder verdreht werden, dass sich zudem die Reibungswirkung zwischen Bowdenzugseil und -mantel derart erhöht, so dass die normale Länge des Bowdenzugseils 13 nicht ausreicht um den distalen Mechanismus in Schubrichtung in seine Endposition zu bringen. In dieser Stellung füllt das Bowdenzugseil 13 durch extreme Krümmung des Bowdenzugs jegliches vorhandene Spiel im Bowdenzugmantel.

Um eine volle Bewegungsfreiheit auch in dieser extremen Stellung zu gewährleisten, kann der Schnellverschluss über seine normale Endposition 15" hinaus - durch Aufwendung einer gegenüber im Normalfall notwendigen größeren Kraft - von einer Bedienperson geschwenkt werden, wobei das Bowdenzugseil 13 trotz Festklemmung um den Betrag dieses Übertretens der Endposition 15" aus der Befestigung im Loch 27 herausrutscht. Ein derartiges Herausrutschen bzw. Durchrutschen kann dabei durch Einstellung einer entsprechenden Festklemmkraft an der Rückholfeder und/oder durch das Zusammenspiel Nocken und Hebel 21 und der daraus resultierenden Überlappung des Lochs 27 ermöglicht werden, ohne dass die Funktion des Bowdenzugs, etwa durch unerwünschtes Verrutschen des Seils 13 im Schnellverschluss 3, eingeschränkt wird.

Hierdurch kann in den Bowdenzug noch mehr Vorspannung bzw. Seillänge eingebracht werden, so dass auch in diesen Extremstellungen des Endoskopschaftes und damit des innenliegenden Bowdenzugs die volle Bewegungsfreiheit des Mechanismus am distalen Ende gewährleistet ist.

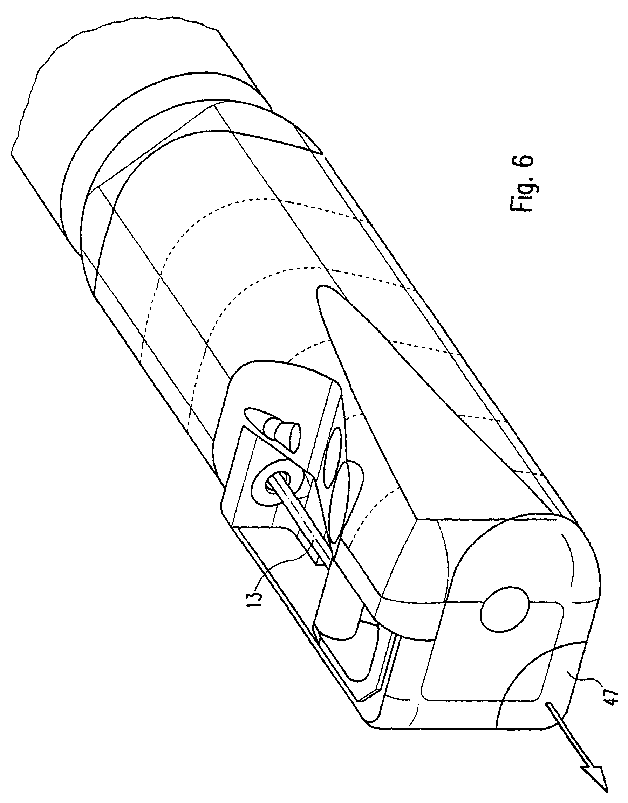

In Fig. 6 und Fig. 7 ist dargestellt, wie ein Mechanismus in einer Trägervorrichtung 39 am distalen Ende des Endokops vom Endoskopkopf abgenommen werden kann.

Hierbei ist nur ein Schnellverschluss über eine Taste 47 am Endoskopkopf, beispielsweise an der Stirnseite in einem Eckbereich der Trägervorrichtung von einer Bedienperson zu betätigen, nachdem der Schnellverschluss 3 am proximalen Ende in Freigabestellung für das Bowdenzugseil 13 gebracht wurde. Der Schnellverschluss am distalen Ende kann hierbei beispielsweise in Form einer Ausnehmung des Endoskopkopfes ausgebildet sein, in welche ein von einer Bedienperson betätigbares hierzu komplementäres Eingriffselement in der Trägervorrichtung 39 in der in Fig. 6 dargestellten Position eingreift. Wie in Fig. 7 dargestellt, kann diese Ausnehmung als eine, beispielsweise umlaufende, Rille 51 in einem in Längsrichtung herausragenden Zapfen 49 ausgebildet sein, so dass dieser Zapfen 49 in eine entsprechende Ausnehmung in der Trägervorrichtung 39 eingreifen kann. Bei aufgesetzter Trägervorrichtung (Fig. 6) greift in einer Ebene senkrecht zur Längsachse des Zapfens 49 ein über die Taste 47 von einer Bedienperson von außen betätigbares Eingriffselement 53 in diese Rille 51 ein, so dass die Trägervorrichtung 39 fest am Endoskopkopf fixiert ist. Das Eingriffselement 53 kann hierfür beispielsweise als Lochplatte ausgebildet sein, welche durch Federkraft in Eingriffsrichtung gedrückt bwz. vorgespannt ist, so dass ein Teil des Lochrandes in die Rille 51 eingreift, und über die Taste 47 aus dieser Eingriffsstellung ausgelenkt werden kann.

Wie in Fig. 7 ersichtlich kann bei abgenommener Trägervorrichtung 39 dann bei Vorhandensein eines Bowdenzugkanals der Bowdenzugmantel ebenfalls wie das Bowdenzugseil 13 am distalen Ende des Endoskops zumindest ein Stück oder ganz herausgezogen werden. Wie in Fig. 7 dargestellt, bildet die dem Endoskopkopf zugewandte Rückwand der Trägervorrichtung 39 gegenüber dem distalen Ende des Bowdenzugmantels einen Anschlag in Schubrichtung. Hierdurch kann die notwendige Verbindung zwischen Endoskopmantel und Endoskop, um den Bowdenzugmantel in Längsrichtung zu fixieren, abgesehen von einer festen Schraubverbindung, auch durch das einfache Einsetzen der Trägervorrichtung 39 erreicht werden, wobei der Bowdenzugmantel am proximalen Ende durch einen nicht näher dargestellten Anschlag in Zugrichtung und durch diese Rückwand der eingesetzten Trägervorrichtung 39 am am distalen Ende in Schubrichtung begrenzt und damit in seiner Längsrichtung sicher fixiert ist.

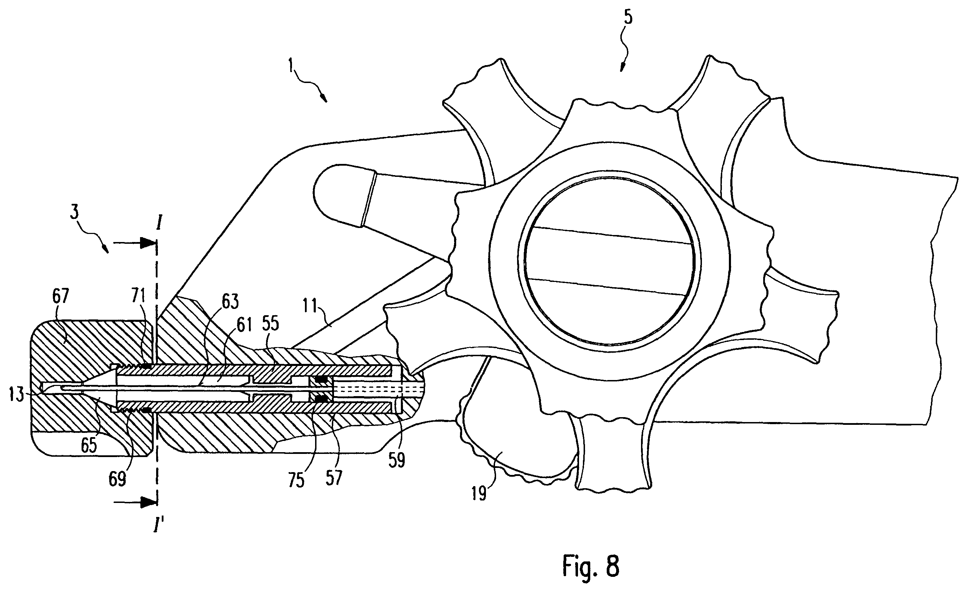

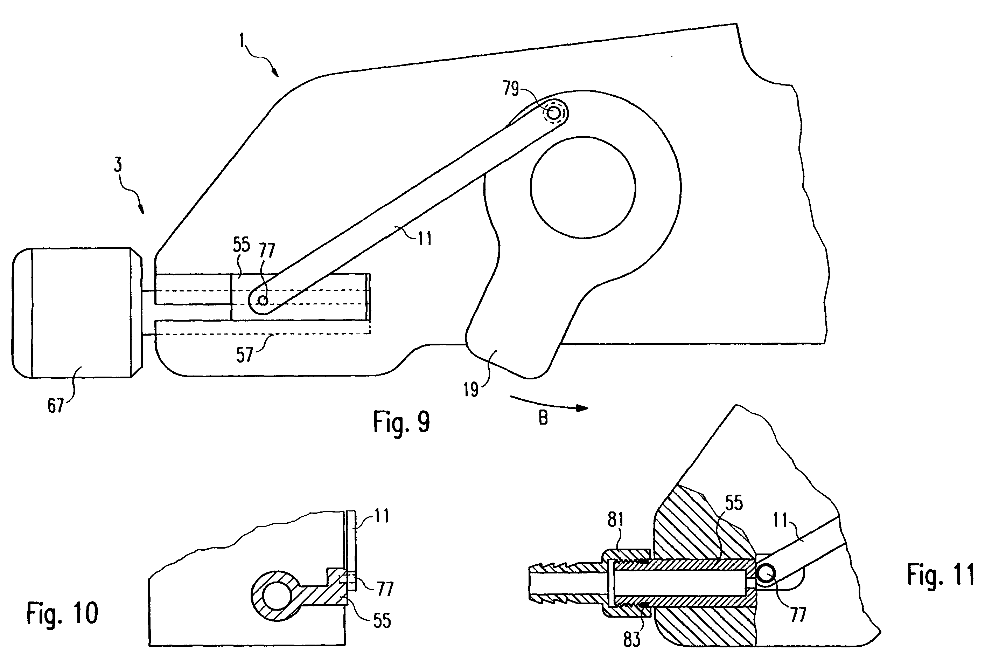

Fig. 8 zeigt eine andere Ausführungsform nach der Erfindung, bei der die Schwenkbewegung des Betätigungshebels 19 über den Hebel bzw. die Stange 11 in eine Bewegung in Längsrichtung zum Bowdenzugseil 13 umgesetzt wird. Hierzu ist der Hebel 11 an seinem einen Ende, wie in Fig. 9 ersichtlich, am Betätigungshebel 19 beispielsweise mittels einer Achse 79 bzw. einem Bolzen exzentrisch um eine Achse senkrecht zur Zeichenebene drehbar gelagert und an seinem anderen Ende beispielsweise mittels einer Schraube 77 an einem nach außen ragenden Teilbereich eines Schlittens 55 um eine Achse senkrecht zur Zeichenebene drehbar gelagert. Hierdurch wird der Schlitten 55 bei einer Drehung des Hebels 19 in Richtung B in Längsrichtung des Bowdenzugseils 13 aus dem proximalen Ende 3 des Endoskops herausbewegt und bei einer entgegengesetzten Betätigung des Hebels 19 hineinbewegt.

Der maximale Bewegungs- bzw. Arbeitsbereich des Schlittens 55 kann hierbei durch entsprechende Anordnung der Exzenterstange 11 bzw. durch den Abstand ihrer Lagerung 79 von der Drehachse des Betätigungshebels 19 größer als der eigentliche Bewegungsbereich des Bowdenzugseils bzw. des hiermit am distalen Ende des Endoskops verbundenen Mechanismus gewählt werden. Hierdurch lässt sich ebenso, wie vorstehend für die erste Ausführungsform beschrieben, eine Vorspannung in den Bowdenzug einbringen.

In der dargestellten zweiten Ausführungsform ist der in Längsrichtung des Bowdenzugseils 13 schwenkbare bzw. verschiebbare Schnellverschluss 3 mit einer Spannzange 61 augerüstet, die mittels eines Spannelements beispielsweise in Form einer Spannmutter 67 gespannt werden kann, um das in der Spannzange 61 geführte Ende des Bowdenzugseils 13 in Längsrichtung ortsfest im Schlitten 55 zu fixieren.

Wie in Fig. 8 dargestellt, kann der Schlitten 55 im Wesentlichen zylinderförmig ausgebildet sein und an seinem Außenumfang in Längsrichtung zumindest über einen Teil seiner Länge einen schlüsselbartförmigen Vorsprung aufweisen. Dieser Schlitten 55 befindet sich in einer am proximalen Ende des Endoskops in Längsrichtung des Bowdenzugseils 13 angeordneten geschlitzten Bohrung 63, deren Innenabmessungen im Querschnitt komplementär bzw. geringfügig größer zu den Außenabmessungen des Schlittens 55 ausgebildet sind, so dass sich der Schlitten 55 in dieser Bohrung 57 in Längsrichtung zum Bowdenzugseil 13 leichtgängig bewegen lässt.

Zur Aufnahme des Bowdenzugseils 13 weist der zylinderförmige Bereich des Schlittens 55 in seinem in distale Richtung weisenden Ende eine Ausnehmung beispielsweise in Form einer Bohrung auf, deren Innenabmessungen groß genug sind, um das Ende einer Bowdenzugvorrichtung, also beispielsweise einen zylinderförmigen Gehäusevorsprung der als Anschlag oder als Befestigung für einen Bowdenzugmantel dient, aufzunehmen. Um eine eventuell wünschenswerte Dichtwirkung zu ermöglichen kann zwischen der Innenwandung der Bohrung des Schlittens 55 und dem Ende der Bowdenzugvorrichtung eine Dichtung, beispielsweise in Form eines O-Rings, angeordnet sein, die wahlweise ortsfest in einer ringförmigen Ausnehmung der Bohrung des Schlittens 55 oder, wie in Fig. 8 dargestellt, in einer ringförmigen Ausnehmung am Ende der Bowdenzugvorrichtung sitzt. Am anderen am proximalen Ende 3 des Endoskops herausragenden Ende des Schlittens 55 befindet sich eine Ausnehmung, beispielsweise in Form einer Bohrung, in Längsrichtung des Bowdenszugseils 13 als Aufnahme für eine Spannzange 61. Diese Bohrung kann, wie im Beispiel dargestellt, in ihrer Tiefe begrenzt sein, so dass der Schlitten 55 einen Mittelbereich mit einer zu diesen Bohrungen im Durchmesser kleineren Bohrung aufweist, die zumindest einen geringfügig größeren Durchmesser als der Außendurchmesser des Bowdenzugseils 13 aufweist.

Hierdurch ist es möglich, den Schlitten 55 in die Schlittenaufnahme 57 am proximalen Ende 3 des Endoskops einzusetzen, wobei das proximale Ende des Bowdenzugseils 13 sich durch die Ausnehmungen in Längsrichtung im Inneren des Schlittens 55 hindurchschiebt und zumindest zu einem Teil in der Bohrung am proximalen Ende des Schlittens 55 hineinragt. Zur Befestigung des Seilendes wird in diese Bohrung die Spannzange 61 eingesetzt, die in ihrer zentralen Bohrung 63 das Ende des Bowdenzugseils 13 aufnimmt. Als Einführungshilfe für das Bowdenzugseilende kann die Spannzange 61 an ihrer in distale Richtung weisende Öffnung eine kegelförmige Erweiterung aufweisen. Am proximalen Ende weist die im Wesentlichen zylinderförmig ausgebildete Spannzange 61 einen kegelförmigen Kopf 65 mit beispielsweise vier Längsschlitzen auf. Wird dieser kegelförmige Kopf 65 von außen mit einer Kraft beaufschlagt, so ermöglichen die Schlitze eine Verkleinerung der inneren Bohrung und somit ein Einklemmen des darin befindlichen Abschnittes des Bowdenzugseils 13. Die Krafteinwirkung kann, wie im Ausführungsbeispiel dargestellt, durch ein Spannelement beispielsweise in Form einer Spannmutter 67 erfolgen, die eine Bohrung mit einem konusförmigen Ende bzw. Boden aufweist, so dass sich bei einem Aufschieben dieser Spannmutter in Längsrichtung des Bowdenzugseils 13 auf den kegelförmigen Kopf 65 der Spannzange 61 die Spannzange um das Ende des Bowdenzugseils 13 schließt. Als Begrenzungsanschlag entgegen dieser Krafteinwirkung in distaler Richtung dient der stirnseitige Rand der proximalen Öffnung des Schlittens 55, der eine umlaufende Schulter des Kegelkopfs 65 hintergreift. Ebenso wäre es aber denkbar, dass der Begrenzungsanschlag zwischen dem den kegelförmigen Kopf 65 gegenüberliegenden Ende der Spannzange 61 und dem Boden der Bohrung am proximalen Ende des Schlittens 55 gebildet wird.

Wie in Fig. 8 dargestellt, ist die Länge des herausragenden Bowdenzugseilendes so zu wählen, dass es mindestens von der Spannzange 61 fest umschlossen werden kann und andererseits nur soweit aus der Spannzange herausragt, dass sich die Spannmutter 67 ohne verquetschen des Seilendes aufschrauben lässt. Hierzu kann die Spannmutter 67 eine über den konusförmigen Boden hinausreichende Ausnehmung in Form einer Bohrung aufweisen, um einen größeren Aufnahmebereich für das Bowdenzugseilende zu bieten. Bei einer erwünschten Dichtheit zwischen Bowdenzugseils 13 und Bowdenzugmantel durchbricht diese Bohrung, wie in Fig. 8 dargstellt, nicht die Spannmutter 67, wobei der Gewindebereich zwischen Innengewinde der Spannmutter 67 und Außengewinde des proximalen Endes des Schlittens 55 eine Dichtung beispielsweise in Form eines O-Rings 71 sitzen kann.

Durch diese Ausführungsform kann ebenso wie in der ersten Ausführungsform eine Vorspannung in den Bowdenzug eingebracht werden, wobei eine Änderung der Vorspannung und damit des durch die Endpositionen 15', 15" definierten Arbeitsbereiches selbtsverständlich auch während eines Arbeitsvorgangs möglich ist. Hierzu muss nur die Befestigungseinrichtung im Schnellverschluss 3, also die Spannzange 61 durch Lösen der Spannmutter 67 bzw. der Hebel 21 durch Betätigung des Drehknopfs 29 gelöst werden und in einer von dieser Postition unterschiedlichen Feststellposition innerhalb oder außerhalb des vorherigen Arbeitsbereiches durch Aufschrauben der Spannmutter 67 bzw. Senken des Hebel 21 durch Betätigung des Drehknopfs 29 wieder fixiert werden.

Durch diese Veränderung des bereits vorstehend beschriebenen Arbeitsbereiches ergibt sich in der Praxis eine Art Nachfassen des Bowdenzugseils 13, welches in beiden Richtungen erfolgen kann, so dass auch in Extremsituationen, in denen sich die Lage und somit die effektive Länge des Bowdenzugseils 13 im Endoskop durch extreme Verwindung, Krümmung, Verbiegung usw. des Schaftes und/oder des Endoskopkopfes verändert, entsprechend durch Veränderung bzw. Anpassung des neuen Arbeitsbereiches reagiert werden kann.

Fig. 11 zeigt, wie auf das proximal herausragende Ende des Schlittens 55 zu Reinigungszwecken eine Spülkappe 81 aufgesetzt beispielsweise aufgeschraubt werden kann, wobei wiederum eine Dichtung 83 zwischen Innengewinde der Spülkappe und Außengewinde des Schlittenendes für die für einen aureichenden Spüldruck nötige Dichtheit sorgt. Hierdurch kann ebenso wie bei der ersten Ausführungsform der Bowdenzug und, insbesondere bei Entnahme des gesamten Bowdenzugs, der gesamte Bowdenzugkanal von einer Bedienperson beispielsweise von einem Arzt oder einem/einer Arzthelfer/in hervorragend gereingt werden ohne einen kostenintsensiven und zeitaufwendigen Wartungsdienst oder Techniker zu bemühen.

Selbstverständlich sind anstatt der dargestellten Befestigungsart bzw. der dargestellten Spannzange 61 auch andere in Längsrichtung des Bowdenzugseils 13 verschiebbare Befestigungsarten denkbar, beispielsweise eine in distaler Richtung kegelförmig ausgebildete Spannzange, die in eine konusförmige Bohrung am proximalen Ende des Schlittens 55 eingedrückt wird. Ebenso sind statt einer Spannmutter 67 auch andere Spannelemente wie einrastende Hebel, Federn oder ähnliches denkbar, welche die Spannzange in entsprechender Richtung mit einer die Spannzange schließenden Kraft beaufschlagen.

## Patentansprüche

1. Flexibles Endoskop mit einem am proximalen Ende schwenkbar oder verschiebbar angeordneten ersten Schnellverschluss (3) für ein Bowdenzugseil (13) eines Bowdenzugs mit einer Befestigungseinrichtung für das Lösen und das Befestigen des Bowdenzugseils (13),
a) wobei der Schnellverschluss (3) mittels der Befestigungseinrichtung mit dem Bowdenzugseil (13) an einer vorbestimmten gleichbleibenden Feststellposition verbindbar ist und
b) der Schnellverschluss (3) einen durch Endpositionen (15', 15") definierten Arbeitsbereich besitzt, in dem das befestigte Bowdenzugseil innerhalb eines durch den Arbeitsbereich des Schnellverschlusses (3) definierten Arbeitsbereichs verschoben werden kann, um mit dem Bowdenzugseil (13) einen am distalen Ende des Endoskops lösbar angeordneten Mechanismus zu betätigen,
**dadurch gekennzeichnet, dass**
c) der Schnellverschluss (3) mittels der Befestigungseinrichtung auch während eines Arbeitsvorgangs von dem Bowdenzugseils (13) lösbar und in einer von der Feststellposition unterschiedlichen Position mit dem Bowdenzugseils (13) verbindbar ist, um eine Vorspannung in den Bowdenzug einzubringen und insbesondere ein Nachfassen des Bowdenzugseils (13) zu ermöglichen und
d) der Schnellverschluss (3) als in Längsrichtung des Bowdenzugseils verschiebbare Spannvorrichtung ausgebildet ist, welche einen Schlitten (55) mit einer mittels eines Spannelements (67) spannbaren Spannzange (61) umfasst.

2. Flexibles Endoskop mit einem am proximalen Ende schwenkbar oder verschiebbar angeordneten ersten Schnellverschluss (3) für ein Bowdenzugseil (13) eines Bowdenzugs mit einer Befestigungseinrichtung für das Lösen und das Befestigen des Bowdenzugseils (13),
a) wobei der Schnellverschluss (3) mittels der Befestigungseinrichtung mit dem Bowdenzugseil (13) an einer vorbestimmten gleichbleibenden Feststellposition verbindbar ist und
b) der Schnellverschluss (3) einen durch Endpositionen (15', 15") definierten Arbeitsbereich besitzt, in dem das befestigte Bowdenzugseil innerhalb eines durch den Arbeitsbereich des Schnellverschlusses (3) definierten Arbeitsbereichs verschoben werden kann, um mit dem Bowdenzugseil (13) einen am distalen Ende des Endoskops lösbar angeordneten Mechanismus zu betätigen,
**dadurch gekennzeichnet, dass**
c) der Schnellverschluss (3) mittels der Befestigungseinrichtung auch während eines Arbeitsvorgangs von dem Bowdenzugseils (13) lösbar und in einer von der Feststellposition unterschiedlichen Position mit dem Bowdenzugseils (13) verbindbar ist, um eine Vorspannung in den Bowdenzug einzubringen und insbesondere ein Nachfassen des Bowdenzugseils (13) zu ermöglichen und
d) die Befestigungseinrichtung als eine Klemmeinrichtung ausgebildet ist, die auf das Bowdenzugseil (13) mittels eines vorgespannten Hebels (21) eine vorbestimmte Klemmkraft ausübt.

3. Endoskop nach Anspruch 1 oder 2, **dadurch gekennzeichnet, dass** die Feststellposition sich in einer von den Endpositionen (15', 15") entfernten Lage befindet, um in den Bowdenzug eine Vorspannung einzubringen.

4. Endoskop nach Anspruch 2 oder 3 wenn abhängig von Anspruch 2, **dadurch gekennzeichnet, dass** der Schnellverschluss (3) ein Bedienelement für ein Arretieren des Schnellverschlusses (3) in der Feststellposition bei gleichzeitigem Betätigen der Befestigungseinrichtung aufweist.

5. Endoskop nach Anspruch 4, **dadurch gekennzeichnet, dass** das Bedienelement als Drehknopf (29) ausgebildet ist, welcher für das Betätigen der Befestigungseinrichtung einen Nocken (33) aufweist.

6. Endoskop nach Anspruch 4 oder 5, **dadurch gekennzeichnet, dass** das Bedienelement über einen Bereich seines Außenumfangs eine Ausnehmung in Form einer Schnecke (35) aufweist, in die ein am Endoskopgehäuse ortsfest angeordnetes Eingriffselement (49) eingreifen kann.

7. Endoskop nach einem der vorhergehenden Ansprüche, **dadurch gekennzeichnet, dass** der am distalen Ende des Endoskops angeordnete Mechanismus mittels eines von außen betätigbaren zweiten Schnellverschlusses am distalen Ende des Endoskops lösbar angeordnet ist.

8. Endoskop nach Anspruch 7, **dadurch gekennzeichnet, dass** der zweite Schnellverschluss als Schnapp- oder Rastverschluß ausgebildet ist.

9. Endoskop nach Anspruch 7 oder 8, **dadurch gekennzeichnet, dass** der Mechanismus am distalen Ende in einer Trägervorrichtung (39) angeordnet ist, die mittels des Schnellverschlusses lösbar am Endoskopkopf angeordnet ist und als distaler Begrenzungsanschlag für einen Bowdenzugmantel dient, so dass nach dem Lösen des ersten und zweiten Schnellverschlusses am distalen und proximale Ende des Endoskops und der Abnahme der Trägervorrichtung (39) der in einem Bowdenzugkanal verlaufende Bowdenzugmantel in distaler Richtung längsverschiebbar ist und am distalen Ende durch eine Bedienperson entnehmbar ist.

10. Endoskop nach einem der vorhergehenden Ansprüche, **dadurch gekennzeichnet, dass** der erste Schnellverschluss (3) über seine Endposition (15") in Zugrichtung durch Aufwendung einer gegenüber der in Arbeitsfunktion nötigen höheren Kraft hinausbewegbar ist, so dass das proximale Ende des Bowdenzugseils (13) um den Betrag dieser Hinausbewegung in der Befestigungseinrichtung durchrutscht.

## Claims

1. Flexible endoscope having a first quick-action closing element (3), arranged in a pivoted or displaceable manner at the proximal end, for a Bowden control cable (13) of a Bowden control having a securing device for detaching and securing the Bowden control cable (13),
a) wherein the quick-action closing element (3) can be connected to the Bowden control cable (13) by means of the securing device in a predetermined constant fixing position, and
b) the quick-action closing element (3) has a working area that is defined by end positions (15', 15'') and in which the secured Bowden control cable can be displaced within a working area defined by the working area of the quick-action closing element (3) in order to actuate, by means of the Bowden control cable (13), a mechanism that is detachably arranged at the distal end of the endoscope,
**characterised in that**
c) by means of the securing device even during a work operation the quick-action closing element (3) can be detached from the Bowden control cable (13) and connected to the Bowden control cable (13) in a position that is different from the fixing position in order to introduce a pretension into the Bowden control and in particular in order to enable the Bowden control cable (13) to be regripped, and
d) the quick-action closing element (3) is formed as a tensioning arrangement which can be displaced in the longitudinal direction of the Bowden control cable and comprises a slide (55) with a collet chuck (61) that can be tensioned by means of a tensioning element (67).

2. Flexible endoscope having a first quick-action closing element (3), arranged in a pivoted or displaceable manner at the proximal end, for a Bowden control cable (13) of a Bowden control having a securing device for detaching and securing the Bowden control cable (13),
a) wherein the quick-action closing element (3) can be connected to the Bowden control cable (13) by means of the securing device in a predetermined constant fixing position, and
b) the quick-action closing element (3) has a working area that is defined by end positions (15', 15'') and in which the secured Bowden control cable can be displaced within a working area defined by the working area of the quick-action closing element (3) in order to actuate, by means of the Bowden control cable (13), a mechanism that is detachably arranged at the distal end of the endoscope,
**characterised in that**
c) by means of the securing device even during a work operation the quick-action closing element (3) can be detached from the Bowden control cable (13) and connected to the Bowden control cable (13) in a position that is different from the fixing position in order to introduce a pretension into the Bowden control and in particular in order to enable the Bowden control cable (13) to be regripped, and
d) the securing device is formed as a clamping device which exerts a predetermined clamping force on the Bowden control cable (13) by means of a pretensioned lever (21).

3. Endoscope according to claim 1 or 2, **characterised in that** the fixing position is in a location that is remote from the end positions (15', 15'') in order to introduce a pretension into the Bowden control.

4. Endoscope according to claim 2 or 3, when dependent on claim 2, **characterised in that** the quick-action closing element (3) has an operating element for arresting the quick-action closing element (3) in the fixing position with simultaneous actuation of the securing device.

5. Endoscope according to claim 4, **characterised in that** the operating element is formed as a turning knob (29) which has a cam (33) for the actuation of the securing device.

6. Endoscope according to claim 4 or 5, **characterised in that** the operating element has, over a region of its outer periphery, a recess in the form of a helix (35) into which an engaging element (49) that is arranged in a stationary manner on the endoscope housing can engage.

7. Endoscope according to one of the preceding claims, **characterised in that** the mechanism that is arranged at the distal end of the endoscope is detachably arranged at the distal end of the endoscope by means of a second quick-action closing element that can be actuated from without.

8. Endoscope according to claim 7, **characterised in that** the second quick-action closing element is formed as a snap-action or latching closing element.

9. Endoscope according to claim 7 or 8, **characterised in that** the mechanism is arranged at the distal end in a carrier arrangements(39) which is detachably arranged on the endoscope head by means of the quick-action closing element and serves as a distal limiting stop for a Bowden control casing so that after detachment of the'first and second quick-action closing element at. the distal and proximal end of the endoscope and the removal of the carrier arrangement (39) the Bowden control casing extending in a Bowden control channel can be longitudinally displaced in the distal direction and can be taken out at the distal end by an operator.

10. Endoscope according to one of the preceding claims, **characterised in that** the first quick-action closing element (3) can be moved beyond its end position (15'') in the direction of pull by means of the application of a force that is higher than that required in the work function so that the proximal end of the Bowden control cable (13) slips through in the securing device by the amount of this overtravel.

## Revendications

1. Endoscope flexible comprenant une première fermeture rapide (3), agencée avec faculté de pivotement ou de translation à une extrémité proximale, pour un câble (13) d'une transmission Bowden avec un dispositif de fixation pour le détachement et la fixation du câble (13) Bowden,
a) la fermeture rapide (3) est susceptible d'être reliée à une position d'immobilisation prédéterminée constante au moyen du dispositif de fixation avec le câble (13) Bowden,
b) la fermeture rapide (3) possède une zone de travail définie par des positions finales (15', 15"), dans laquelle le câble Bowden fixé peut être déplacé à l'intérieur d'une zone de travail définie par la zone de travail de la fermeture rapide (3) pour actionner avec le câble Bowden (13) un mécanisme agencé de manière détachable à l'extrémité distale de l'endoscope,
**caractérisé en ce que**
c) la fermeture rapide (3) est susceptible d'être reliée au câble Bowden (13) au moyen du dispositif de fixation d'une manière susceptible d'être détachée du câble Bowden (13) également pendant un cycle de travail et dans une position différente de la position d'immobilisation afin d'introduire dans la transmission Bowden une précontrainte et de permettre en particulier une reprise du câble Bowden (13), et
d) la fermeture rapide (3) est réalisé sous forme d'un dispositif de tensionnement susceptible d'être déplacé dans la direction longitudinale du câble Bowden et qui comprend un chariot (55) avec une pince de tensionnement (61) susceptible d'être mise sous tension au moyen d'un élément de tensionnement (67).

2. Endoscope flexible comprenant une première fermeture rapide (3), agencée avec faculté de pivotement ou de translation à une extrémité proximale, pour un câble (13) d'une transmission Bowden avec un dispositif de fixation pour le détachement et la fixation du câble (13) Bowden,
a) dans lequel la fermeture rapide (3) est susceptible d'être reliée à une position d'immobilisation prédéterminée constante avec le câble (13) Bowden, et
b) la fermeture rapide (3) possède une zone de travail définie par des positions finales (15', 15"), dans laquelle le câble Bowden fixé peut être déplacé dans une zone de travail définie par la zone de travail de la fermeture rapide (3) afin d'actionner avec le câble Bowden (13) un mécanisme agencé de manière détachable à l'extrémité distale de l'endoscope,
**caractérisé en ce que**
c) la fermeture rapide (3) est susceptible d'être reliée au câble Bowden (13) au moyen du dispositif de fixation d'une manière susceptible d'être détachée du câble Bowden (13) également pendant un cycle de travail et dans une position différente de la position d'immobilisation afin d'introduire dans la transmission Bowden une précontrainte et de permettre en particulier une reprise du câble Bowden (13), et
d) le dispositif de fixation est réalisé à la manière d'un dispositif de pincement qui exerce sur le câble Bowden (13) une force de pincement prédéterminée au moyen d'un levier (21) sous précontrainte.

3. Endoscope selon l'une ou l'autre des revendications 1 et 2, **caractérisé en ce que** la position d'immobilisation se trouve dans une position éloignée des positions finales (15', 15") afin d'introduire une précontrainte dans la transmission Bowden.

4. Endoscope selon la revendication 2, ou selon la revendication 3 prise en dépendance de la revendication 2, **caractérisé en ce que** la fermeture rapide (3) comprend un élément de manipulation pour arrêter la fermeture rapide (3) dans la position d'immobilisation lors d'un actionnement simultané du dispositif de fixation.

5. Endoscope selon la revendication 4, **caractérisé en ce que** l'élément de manipulation est réalisé sous forme de bouton tournant (29), lequel comporte une came (33) pour l'actionnement du dispositif de fixation.

6. Endoscope selon l'une ou l'autre des revendications 4 et 5, **caractérisé en ce que** l'élément de manipulation comporte, sur une zone de sa périphérie extérieure, un évidement sous la forme d'un escargot (35), dans lequel peut s'engager un élément d'engagement (49) agencé de manière stationnaire sur le boîtier de l'endoscope.

7. Endoscope selon l'une des revendications précédentes, **caractérisé en ce que** le mécanisme agencé à l'extrémité distale de l'endoscope est agencé de manière détachable à l'extrémité distale de l'endoscope au moyen d'une seconde fermeture rapide susceptible d'être actionnée depuis l'extérieur.

8. Endoscope selon la revendication 7, **caractérisé en ce que** la seconde fermeture rapide est réalisée sous forme de fermeture à encliquetage ou de fermeture à enclenchement.

9. Endoscope selon l'une ou l'autre des revendications 7 et 8, **caractérisé en ce que** le mécanisme à l'extrémité distale est agencé dans un dispositif support (39) qui est agencé de manière détachable sur la tête de l'endoscope au moyen de la fermeture rapide et sert de butée de délimitation distale pour une gaine de transmission Bowden, de sorte qu'après le détachement de la première et de la seconde fermeture rapide à l'extrémité distale et à l'extrémité proximale de l'endoscope et enlèvement du dispositif support (39) la gaine Bowden qui s'étend dans un canal de transmission Bowden est susceptible d'être déplacée longitudinalement en direction distale et d'être enlevée à l'extrémité distale par un opérateur.

10. Endoscope selon l'une des revendications précédentes, **caractérisé en ce que** la première fermeture rapide (3) est susceptible d'être déplacée au-delà de sa position finale (15") en direction de traction en appliquant une force plus élevée que la force nécessaire dans la fonction de travail, de sorte que l'extrémité proximale du câble Bowden (13) patine dans le dispositif de fixation de l'amplitude de ce déplacement vers l'extérieur.
